# EUROPEAN PATENT APPLICATION

(11) **EP 4 302 769 A1**
(43) Date of publication of application: **10.01.2024**
(21) Application number: 22791424.9
(22) Date of filing: 16.03.2022
(51) Int. Cl.: A61K 35/20, A61K 8/98, A61P 17/04, A61P 29/00, A61P 37/08, A61P 43/00, A61Q 19/00, A61K 8/9728, A61K 36/06

(54) **KUMIS-DERIVED EXOSOME AND USE THEREOF**

(30) Priority: 19.04.2021 JP 2021070665
(71) Applicant: Eerdeng Bulude, Tokyo 173-0003 (JP)
(72) Inventor: OCHIYA Takahiro, Tokyo 104-0053 (JP); EERDENG Bulude, Tokyo 173-0003 (JP)
(74) Representative: Schön, Christoph
(86) International application number: PCT/JP2022/011880
(87) International publication number: WO 2022/224644

(57) **Abstract**

A new material that can suppress TARC, related to the onset of various inflammatory diseases, and is useful, for example, for the treatment of atopic dermatitis, and the like is provided. The present invention is a kumis-derived exosome-containing composition including an exosome-enriched fraction obtained from kumis and enriched with exosomes derived from the kumis. The present invention is a method for producing a kumis-derived exosome-containing composition, having a step of fractionating kumis into multiple fractions and a step of collecting a fraction enriched with exosomes from the multiple fractions. This kumis-derived exosomes have the function and effect of suppressing TARC production, and are useful as a material to be blended in a composition for treating skin or an anti-inflammatory composition. The exosomes are also useful as an agent for treating TARC-mediated disease such as atopic dermatitis.

## Description

### Technical Field

The present invention relates exosomes derived from kumis and the use thereof.

### Background Art

Exosomes are extracellular vesicles secreted by cells and having sizes of 30 to 200 nm (nanometers). Membrane proteins, intracellular proteins, nucleic acids (microRNAs, messenger RNAs, DNAs, and the like), and the like, which are informational molecules comprised by cells, are incorporated therein. It is believed that exosomes transmit information to other cells (Non Patent Literature 1). In recent years, the applications of these exosomes as medical supplies, cosmetics, functional foods, and the like have also been energetically attempted.

With respect to the application of exosomes to medical supplies, cosmetics, and the like, for example, Patent Literature 1 discloses invention as to a pharmaceutical composition containing exosomes derived from mesenchymal stem cells and the use thereof in a method for treating immune-mediated inflammatory disease. Patent Literature 2 discloses invention as to an anti-inflammatory agent containing exosomes derived from milk as an active ingredient. Patent Literature 3 discloses invention as to exosomes extracted from yeast mash for sake or unrefined sake, or miso, and states that exosomes derived from yeast (yeast mash, unrefined sake, or miso) increases the expression levels of granzymes, which are cytocidal factors secreted from NK cells and that the addition of yeast mash exosomes increases the gene expression levels of collagen and elastin in senescent fibroblasts (paragraph 0055 of Patent Literature 3).

Meanwhile, TARC (thymus and activation-regulated chemokine) is known as one of chemokines that exhibit chemotaxis and the like of leukocytes. For example, it is believed that, in atopic dermatitis, TRAC produced by epidermal keratinocytes in lesions causes lymphocytes (Th2 cells that express CCR4) to migrate locally, and Th2-dominant immune response leads to IgE production and the infiltration and activation of eosinophils to result in allergic symptoms (Non Patent Literature 2).

For example, Patent Literature 4 discloses invention as to a human CCL17 antibody, and states that since CCL17 ("CCL17" is a synonym for "TARC") is related to human diseases such as ulcerative colitis, atopic dermatitis, idiopathic pulmonary fibrosis, and asthma that influence various organs, the human CCL17 antibody is useful for the treatment of such CCL17-mediated inflammatory diseases (paragraphs 0004 and 0144 of Patent Literature 4).

### Citation List

### Non Patent Literature

Non Patent Literature 1
   Kosaka N, Yoshioka Y, Fujita Y, Ochiya T. "Versatile roles of extracellular vesicles in cancer." J Clin Invest. 2016 Apr 1; 126(4), pp1163-72.
Non Patent Literature 2
   Yuichiro Tsunemi "Atopi-sei Hihuen Shinryou ni okeru Kessei TARC-ti no Katsuyou (Utilization of Serum TARC Value in Diagnosis and Treatment of Atopic Dermatitis)" Journal of Environmental Dermatology and Cutaneous Allergology 11(3); 2017, pp220-226.

### Patent Literature

Patent Literature 1
   Japanese Translation of PCT International Application Publication No. 2018-531979
Patent Literature 2
   International Publication No. WO 2016/039356
Patent Literature 3
   International Publication No. WO 2020/158930
Patent Literature 4
   Japanese Translation of PCT International Application Publication No. 2016-537024

### Summary of Invention

### Technical Problem

It is believed that if TARC, related to the onset of various inflammatory diseases, can be suppressed, this will be promising as therapeutic drugs and the like for those diseases. It is however difficult in practical view of the form to be administered to a patient, the exhibition of the effect, and the like to say that, for example, the antibody technology described in Patent Literature 4 is versatile.

Accordingly, an object of the present invention is to provide a new material that can suppress TARC related to the onset of various inflammatory diseases, and is useful, for example, for the treatment of atopic dermatitis, and the like.

### Solution to Problem

In order to achieve the above-mentioned object, the present inventors have investigated extensively, consequently found that kumis-derived exosomes have the function and effect of suppressing the production of TARC from normal human epidermal keratinocytes that causes inflammatory response, and completed the present invention.

That is, first, the present invention provides a composition for treating skin, comprising: kumis-derived exosomes.

Second, the present invention provides an anti-inflammatory composition, comprising: kumis-derived exosomes.

Third, the present invention provides a composition for suppressing TARC production, comprising: kumis-derived exosomes.

Fourth, the present invention provides a composition for treating atopic dermatitis, comprising: kumis-derived exosomes.

Fifth, the present invention provides an agent for suppressing TARC production, comprising: kumis-derived exosomes as an active ingredient.

Sixth, the present invention provides a composition for treating disease, comprising: the above-mentioned agent for suppressing TARC production.

It is preferable that, in the above-mentioned composition for treating disease, the disease be a TARC-mediated disease. It is preferable that the disease be atopic dermatitis.

Seventh, the present invention provides a method for producing a kumis-derived exosome-containing composition, comprising: a step of fractionating kumis into multiple fractions; and a step of collecting a fraction enriched with exosomes from the multiple fractions.

Eighth, the present invention provides a kumis-derived exosome-containing composition comprising an exosome-enriched fraction obtained from kumis and enriched with exosomes derived from the kumis.

### Advantageous Effects of Invention

According to kumis-derived exosomes provided by the present invention, TARC, related to the onset of various inflammatory diseases, can be suppressed, and the exosomes are useful, for example, for treatment of atopic dermatitis, and the like. As a blending component of a composition for treatment to be administered to a human or an animal, the exosomes can be suitably used.

### Brief Description of Drawings

FIG. 1 is figures showing the results of measuring kumis-derived exosomes prepared in Preparation Example 1 with a nanoparticle-tracking analysis system. FIG. 1 (a) shows a graph of the particle size distribution, and FIG. 1 (b) shows an example of an observed visual field image.
FIG. 2 is a figure showing the results of inspecting the influence of kumis-derived exosomes on the expression level (relative value) of TARC produced at the time of making IL-10/TNF-α/IFN-γ act on normal human epidermal keratinocytes to cause inflammation in Test Example 1.
FIG. 3 is a figure showing the results of inspecting the influence of kumis-derived exosomes on the expression level (relative value) of TSLP produced at the time of making IL-10/TNF-α/IFN-γ act on normal human epidermal keratinocytes to cause inflammation in Test Example 1.
FIG. 4 is a figure showing the results of inspecting the influence of kumis-derived exosomes on the expression level (relative value) of MDC produced at the time of making IL-10/TNF-α/IFN-γ act on normal human epidermal keratinocytes to cause inflammation in Test Example 1.

### Description of Embodiments

"Kumis" used herein is synonymous with the meaning usually understood by those skilled in the art, and is specifically a beverage obtained by fermenting horse milk naturally, having an alcohol content of 1 to 3%, and containing nutrients such as minerals and vitamins derived from the horse milk. Kumis can be obtained by, for example, adding the remainder of prepared kumis to freshly drawn horse milk for stirring. It is preferable to use kumis produced in the State of Mongolia as the country of origin.

"Exosomes" used herein are synonymous with the meaning usually understood by those skilled in the art, and are specifically extracellular vesicles having sizes of 30 to 200 nm (nanometers) and secreted by cells. Membrane proteins, intracellular proteins, nucleic acids (microRNAs, messenger RNAs, DNAs, and the like), and the like, which are informational molecules comprised by cells, are contained therein.

With respect to the preparation of exosomes from kumis, for example, kumis is treated with acetic acid to precipitate protein for removing protein components abundantly contained in kumis. The supernatant thereof can be separated by ultracentrifugation to obtain highly purified exosomes.

Hereinafter, forms for implementing the present invention will be described in further detail.

### 1. Exosomes derived from kumis

For example, exosomes derived from kumis can be obtained through the following steps:
(a) subjecting kumis to be used as a raw material to centrifugal treatment or filtration treatment to remove impurities;
(b) subjecting the sample from which impurities are removed through the above-mentioned step (a) to ultracentrifugal treatment; and
(c) collecting a precipitate produced by the treatment in the above-mentioned step (b).

Impurities other than exosomes only have to be able to be removed as a precipitate without precipitating the exosomes under the centrifugal force condition in the above-mentioned step (a). For example, the centrifugal force condition may be 10,000 to 20,000 ×g, may be 10,000 to 17,000 ×g, may be 12,000 to 17,000 ×g, or may be 12,000 to 15,000 ×g. For example, the centrifugal time in the above-mentioned step (a) may be 5 to 60 minutes, may be 10 to 45 minutes, or may be 15 to 30 minutes. With respect to the centrifugal temperature in the above-mentioned step (a), the centrifugation can be performed at 4°C to room temperature. Impurities can be further removed from the supernatant obtained by the above-mentioned step (a) by filtration (for example, filtration with a 0.65 µm filter) as needed. The centrifugal treatment or the filtration treatment may be performed in two steps as needed. The centrifugal treatment or the filtration treatment may be performed in random order, or those treatments may be performed alternately.

Exosomes only have to be able to be collected as a precipitate under the centrifugal force condition in the above-mentioned step (b). For example, the centrifugal force condition may be 50,000 to 210,000 ×g, may be 70,000 to 200,000 ×g, may be 70,000 to 200,000 ×g, or may be 100,000 to 180,000 ×g. For example, the centrifugal time in the above-mentioned step (b) may be 50 minutes to 4 hours, may be 60 minutes to 4 hours, or may be 70 minutes to 4 hours. With respect to the centrifugal temperature in the above-mentioned step (b), the centrifugation can be performed at 4°C to room temperature.

In the above-mentioned step (c), the supernatant produced by the ultracentrifugal treatment in the above-mentioned step (b) is removed. A suitable solvent such as phosphate buffered saline (PBS) was added to the precipitate produced by the ultracentrifugal treatment in the above-mentioned step (b), followed by suspension to collect the precipitate. The collected exosomes can be washed with PBS or the like as needed. The suspension of the precipitate in PBS before the repeat of the ultracentrifugal treatment under the conditions in the above-mentioned step (b) enables washing the exosomes.

In the above-mentioned step (a), kumis that is a raw material is subjected to such centrifugal treatment or filtration treatment that the exosomes are not removed, and impurities are removed to prevent the impurities from being mixed into the exosome fractions enriched in the subsequent step.

In the above-mentioned step (b), exosomes floating in kumis is precipitated by ultracentrifugal treatment, and can be separated from components other than exosomes floating, dispersed, dissolved or the like in kumis.

In the above-mentioned step (c), the precipitated exosomes can be suspended in a suitable solvent such as PBS to be collected.

Exosomes contained in kumis are therefore selectively collected for enrichment through the step (a), the step (b), and the step (c).

Here, the "enrichment" means that the concentration in the prepared kumis-derived exosome-containing composition (for example, the concentration may be based on the number, or may be based on the contents of exosome components such as specific proteins, nucleic acids, and lipids) increases preferably 2 times or more, 3 times or more, 4 times or more, 5 times or more, 6 times or more, 7 times or more, 8 times or more, 9 times or more, 10 times or more, 20 times or more, 30 times or more, 40 times or more, 50 times or more, 60 times or more, 70 times or more, 80 times or more, 90 times or more, 100 times or more, 200 times or more, 300 times or more, 400 times or more, 500 times or more, 600 times or more, 700 times or more, 800 times or more, 900 times or more, or 1,000 times or more relative to the concentration of exosomes contained in the kumis that is a raw material.

For example, the measurement of particles having particle sizes in a range peculiar to exosomes or ELISA measurement using an antibody that specifically recognizes exosomes enables confirming whether intended exosomes are contained in the obtained exosome-enriched fractions or not.
The confirmation method is not limited to these, and for example, measurement can be performed with a nanoparticle tracking analysis system that analyzes particles having particle sizes in a range peculiar to exosomes for the particle size based on the difference in Brownian movement speed between individual particles. A commercial analysis system (trade name "NanoSight", available from Malvern Panalytical Ltd.) or the like can be specifically used. ELISA measurement can be performed with an anti-exosome antibody having cross-reactivity to animal species such as horses and bovines. For example, the commercial measurement kit "milk exosome ELISA kit" (available from Cosmo Bio Co., Ltd.) or the like can be specifically used.

For example, the average particle size of the kumis-derived exosomes provided by the present invention may be 80 to 200 nm, may be 100 to 180 nm, or may be 110 to 160 nm. The peak size may be preferably 70 to 140 nm, may be 80 to 130 nm, or may be 90 to 120 nm.

The present invention is not limited to the above-mentioned embodiment, and various modified embodiments are possible within the gist of the present invention, such embodiments are also included in the scope of the present invention. As mentioned above, since the present invention is intended to prepare an exosome-enriched fraction from kumis and use this, from a viewpoint, the present invention provides the kumis-derived exosome-containing composition containing the exosome-enriched fraction enriched with exosomes derived from kumis. From another viewpoint, the present invention provides a method for producing a kumis-derived exosome-containing composition, having a step of fractionating kumis into multiple fractions and a step of collecting a fraction enriched with exosomes from the multiple fractions.

### 2. Composition (agent) containing kumis-derived exosomes

As shown in Test Example, described below, the kumis-derived exosomes have the function and effect of suppressing the production of TARC produced by normal human epidermal keratinocytes that causes inflammatory response.

In an embodiment, the present invention therefore provides various compositions in which kumis-derived exosomes are blended to be applied to a human or an animal for utilization. Examples include the following compositions:
(a) a composition for treating skin, containing kumis-derived exosomes,
(b) an anti-inflammatory composition, containing kumis-derived exosomes,
(c) a composition for suppressing TARC production, containing kumis-derived exosomes,
(d) a composition for treating atopic dermatitis, containing kumis-derived exosomes,
(e) an agent for suppressing TARC production, containing kumis-derived exosomes as an active ingredient, and
(f) a composition for treating disease, containing the agent for suppressing TARC production, containing kumis-derived exosomes as an active ingredient.

With respect to the composition for treating skin, a base material or a carrier that is pharmaceutically acceptable is added to kumis-derived exosomes as necessary, and the mixture is prepared into, for example, a form such as cream, ointment, lotion, gel, spray, a facial pack agent, a patch, a thick agent, or a liniment by a normal formulation method. This can be prepared into the form of a composition to be used for application to skin and the treatment of skin.

The anti-inflammatory composition can be a composition to be used for, for example, preventing, improving, or treating inflammatory symptoms. The administration form or the administration dosage form is not particularly limited, and may be the same as that of the above-mentioned composition for treating skin, or may be any administration form of a peroral form, an intravenous form, an abdominal form, a percutaneous form, a pulmonary form, a transnasal form, an oral form, an enteral form, and the like. In this case, the anti-inflammatory composition may have a form prepared appropriately with a suitable pharmaceutical base material to be suitable for various administration forms thereof and to contain kumis-derived exosomes by a common formulation method. For example, the administration forms may be forms of a tablet, a powdered drug, solution, powder, granules, a soft capsule, a hard capsule, jelly, a troche, intraoral disintegrator, an injection, an inhalant, a suppository, liniment, and the like.

The composition for suppressing TARC production can be a composition to be used for, for example, suppressing TARC production in desired cells. The administration form or the administration dosage form is not particularly limited, and may be the same as that of the above-mentioned composition for treating skin or anti-inflammatory composition.

The composition for treating atopic dermatitis can be a composition to be used for, for example, preventing, improving, or treating symptoms of atopic dermatitis. The administration form or the administration dosage form is not particularly limited, and may be the same as that of the above-mentioned composition for treating skin or anti-inflammatory composition. It is preferable that the administration form or the administration dosage form be a form to be used for application to skin in affected parts of especially atopic dermatitis and the treatment of the skin.

Meanwhile, the kumis-derived exosomes provided by the present invention can be an agent for suppressing TARC production as a drug substance to be used for suppressing TARC production in desired cells. That is, the kumis-derived exosomes can be an agent for suppressing TARC production, containing kumis-derived exosomes as an active ingredient.

For example, the agent for suppressing TARC production, containing the above-mentioned kumis-derived exosomes as the active ingredient can therefore be a composition to be used for, for example, preventing, improving, or treating symptoms of TARC-mediated disease. The administration form or the administration dosage form is not particularly limited, and may be the same as that of the above-mentioned composition for treating skin or anti-inflammatory composition.

Here, the "TARC-mediated disease" is disease in which TARC participates. Representative examples include atopic dermatitis, and examples include contact dermatitis, eczema nummulare, stasis dermatitis, pompholyx, allergic contact dermatitis, folliculitis, psoriasis, an itch due to the dryness of skin, urticaria, asteatotic eczema, seborrheic eczema, dyshidrotic eczema, eczema nummulare, alopecia areata, pollenosis, asthma, and animal allergies besides. Furthermore, examples of the disease of animals such as dogs and cats include tick and flea-mediated skin disease.

When the above-mentioned various compositions are prepared for use, examples of the content of the exosomes contained in the composition can be, for example, 1 to 100% by mass, 2 to 100% by mass, 3 to 100% by mass, 4 to 100% by mass, 5 to 100% by mass, 10 to 100% by mass, 20 to 100% by mass, 30 to 100% by mass, 40 to 100% by mass, or 50 to 100% by mass in terms of the mass of the prepared exosome-enriched fraction. For example, the content can be 4 × 10⁸ to 1 × 10¹⁴ exosomes/mL, 5 × 10⁸ to 1 × 10¹⁴ exosomes/mL, 1 × 10⁹ to 1 × 10¹⁴ exosomes/mL, 1 × 10¹⁰ to 1 × 10¹⁴ exosomes/mL, 1 × 10¹¹ to 1 × 10¹⁴ exosomes/mL, or 1 × 10¹² to 1 × 10¹⁴ exosomes /mL in terms of the number of the exosomes. In addition, 4 × 10⁸ exosomes in terms of the number of exosomes usually are equivalent to around 20 µg in terms of the mass thereof.

When the kumis-derived exosomes provided by the present invention are administered to a human or an animal, the dose of the exosomes can be appropriately set depending on the form or the object of the above-mentioned composition for using this. For example, when the exosomes are ingested as the exosomes for oral ingestion, an adult can ingest, for example, 0.001 mg to 10 g of exosomes per dose in terms of mass of the prepared exosome-enriched fraction several times a day. For example, an adult can ingest 2 × 10⁷ to 2 × 10¹² exosomes in terms of the number of the exosomes. For example, when the exosomes are applied to skin for treating skin, 0.0001 mg to 0.1 g/cm² of the exosomes can be applied in terms of the mass of the prepared exosome-enriched fraction. For example, 2 × 10⁶ to 2 × 10¹⁰ exosomes/cm² can be applied in terms of the number of the exosomes.

The composition containing kumis-derived exosomes provided by the present invention may be used for, for example, preventing, improving, or treating specific symptoms, or may be applied to a healthy human or a healthy animal. For example, the composition may be used to maintain the health of a healthy human or a health animal.

The composition containing kumis-derived exosomes provided by the present invention can be typically used, for example, in various product forms such as medical supplies, quasi-drugs, functional foods, nutritional supplements, supplements, healthy food, medical supplies for animals, quasi-drugs for animals, functional foods for animals, nutritional supplements for animals, supplements for animals, healthy food for animals, feed, and feed additives. Alternatively, the composition can be used in combination of those products. The composition may be used in combination with various foods and drinks.

In an aspect, the present invention provides a method for administering kumis-derived exosomes to a human or an animal for treatment. Examples include the following method:
(a1) a method for treating skin by administering kumis-derived exosomes to a human or an animal,
(b 1) a method for treating inflammation by administering kumis-derived exosomes to a human or an animal,
(c1) a treatment method for suppressing TARC production, suppressing TSLP production, or suppressing MDC production by administering kumis-derived exosomes to a human or an animal,
(d1) a method for treating atopic dermatitis by administering kumis-derived exosomes to a human or an animal, and
(e1) a method for treating TARC-mediated disease by administering kumis-derived exosomes to a human or an animal

In an aspect, the present invention provides the use of kumis-derived exosomes for the administration to a human or an animal and treatment. Examples include the following use:
(a2) use of kumis-derived exosomes for treating skin,
(b2) use of kumis-derived exosomes for treating inflammation,
(c2) use of kumis-derived exosomes for suppressing TARC production, suppressing TSLP production, or suppressing MDC production,
(d2) use of kumis-derived exosomes for treating atopic dermatitis, and
(e2) use of kumis-derived exosomes for treating TARC-mediated disease.

### Examples

The present invention will be described further specifically by giving the Examples. These Examples do not limit the scope of the present invention.

### [Preparation Example 1] <Preparation of kumis-derived exosomes>

Kumis (country of origin: the State of Mongolia) was provided, and 50 mL thereof was subjected to low-speed centrifugation (2000 ×g, 5 minutes) to remove impurities such as disrupted cells. While the temperature was maintained at 37°C, 500 µL of an acetic acid solution was then added for mixing by inversion. The mixture was then separated with a centrifuge (12,000 ×g, 10 minutes, 4°C), and the supernatant was collected in a new tube. This was separated with an ultracentrifugation apparatus (available form BECKMAN COULTER, 100,000 ×g, 70 minutes, 4°C), and the sedimented fraction thereof was collected. The sediment is dispersed in PBS(-), and the same ultracentrifugation was repeated for washing to remove contaminating protein. This was finally suspended in PB S(-), and used for the following tests.

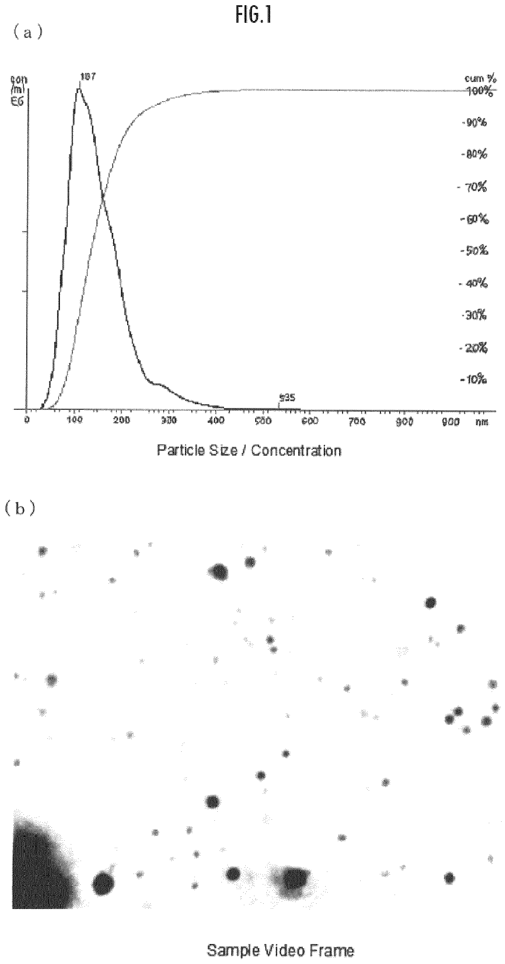

To confirm exosome particles, the end product was evaluated with a nanoparticle tracking analysis system (trade name "NanoSight", available from Malvern Panalytical Ltd.). As shown in FIG. 1, the average of the particle sizes of the kumis exosomes was consequently around 107 nm. The concentration in terms of the concentration contained in kumis that was a raw material was around 4.0 × 10⁸ exosomes/mL. That is, it was revealed that the kumis contains 400,000,000 exosomes per 1 cc.

### (Test Example 1) <Effect in inflammatory model system>

Normal human epidermal keratinocytes (trade name: "Normal Human Epidermal Keratinocytes (NHEKs), adult donor, single donor", PromoCell GmbH) were cultured in a flat bottom plate culture container having 75 cm² in 5% CO₂ at 37°C with culture medium exclusively for keratinocytes (trade name: "Keratinocyte Growth Medium 2 Kit" available from PromoCell GmbH) according to the usual method until subconfluence. The cells were seeded in a new culture container for subculture at such a cell concentration that the cells were 40% confluent. After further culture for 48 hours, the cells were then seeded on a 6-well plate at such a cell concentration that the cells were 80% confluent. After culture for 24 hours, IL-10 was added to a final concentration of 10 ng/mL, TNF-α was added to a final concentration of 10 ng/mL, and IFN-γ was added to a final concentration of 10 ng/mL, followed by further culture for 24 hours. Kumis-derived exosomes was added to culture solution after the culture for 24 hours to a final concentration of 2 µg/mL or 10 µg/mL.

After culture for 48 hours, the cells were collected with a scraper. RNA was collected with a total RNA collection kit (trade name: "NucleoSpin RNA", available from Takara Bio Inc.). A DNA sample for quantifying mRNA was prepared. This sample was subjected to quantitative PCR with specific primers according to the usual method, and the mRNA amounts of TARC (thymus and activation-regulated chemokine), TSLP (thymic stromal lymphopoietin), and MDC (macrophage derived chemokine) were measured. TARC is known as one of chemokines that exhibit chemotaxis and the like of leukocytic cells, and it is known that TARC participates in the onset of atopic dermatitis deeply. It is known that TSLP also participates in selective induction differentiation of Th2 cells (type 2 T-helper cells) and the functional maintenance thereof, and is a master molecule that generally takes part in the occurrence of the allergic inflammation in the same way. MDC (synonym: CCL22) is also known to be one of chemokines that exhibit chemotaxis and the like of leukocytic cells in the same way as TARC and to be a molecule that appears in the serums of atopic dermatitis patients and reflects the severity thereof fully.

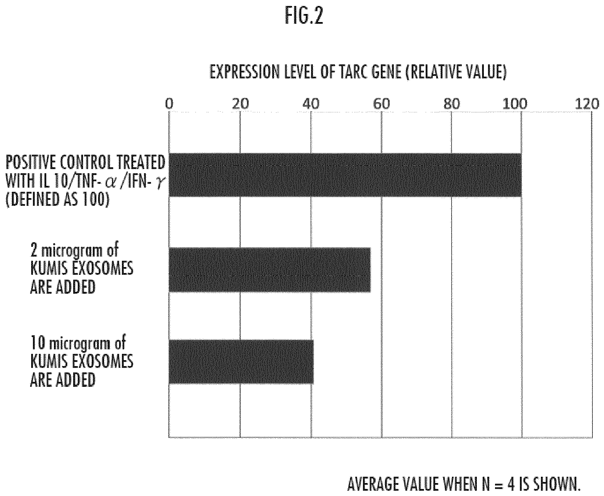

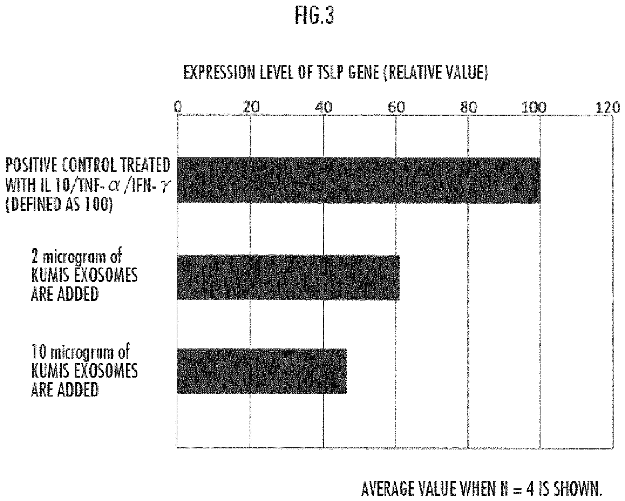

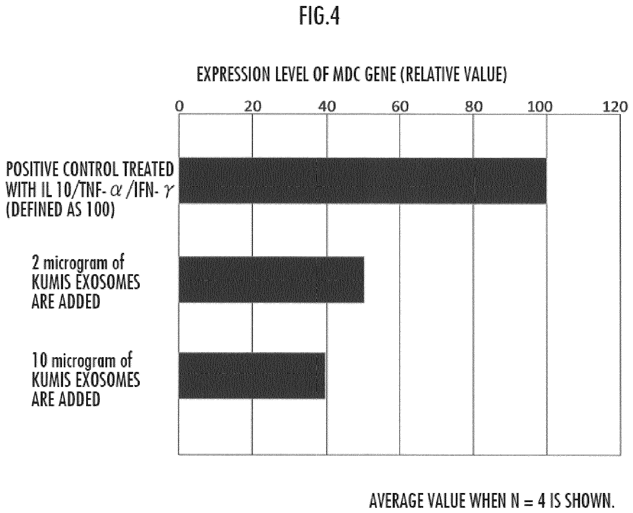

As shown in FIGS. 2, 3, and 4, the expression of TARC, TSLP, and MDC, which were seen to increase in atopic dermatitis patients, was consequently induced by treating normal human epidermal keratinocytes with IL-10/TNF-α/IFN-γ. Meanwhile, the expression induction of these TARC, TSLP, and MDC was significantly suppressed by the addition of the kumis-derived exosomes.

## Claims

1. A composition for treating skin, comprising:
kumis-derived exosomes.

2. An anti-inflammatory composition, comprising:
kumis-derived exosomes.

3. A composition for suppressing TARC production, comprising:
kumis-derived exosomes.

4. A composition for treating atopic dermatitis, comprising:
kumis-derived exosomes.

5. An agent for suppressing TARC production, comprising:
kumis-derived exosomes as an active ingredient.

6. A composition for treating disease, comprising:
the agent for suppressing TARC production according to claim 5.

7. The composition according to claim 6, wherein the disease is TARC-mediated disease.

8. The composition according to claim 6, wherein the disease is atopic dermatitis disease.

9. A method for producing a kumis-derived exosome-containing composition, comprising:
a step of fractionating kumis into multiple fractions; and
a step of collecting a fraction enriched with exosomes from the multiple fractions.

10. A kumis-derived exosome-containing composition, comprising:
an exosome-enriched fraction obtained from kumis and enriched with exosomes derived from the kumis.
